# EUROPEAN PATENT SPECIFICATION

(11) **EP 1 654 270 B1**
(45) Date of publication and mention of the grant of the patent: **26.12.2007**
(21) Application number: 04744169.6
(22) Date of filing: 29.07.2004
(51) Int. Cl.: C07H 19/06, C07H 19/16

(54) **PROCESS FOR PREPARING 2',3'-DIDEHYDRO-2',3'-DIDEOXYNUCLEOSIDES AND 2',3'-DIDEOXYNUCLEOSIDES**
VERFAHREN ZUR HERSELLUNG VON 2',3'-DIDEHYDRO-2',3'-DIDEOXYNUCLEOSIDE UND 2',3'-DIDEOXYNUCLEOSIDE
PROCEDE DE PREPARATION DE 2',3'-DIDEHYDRO-2',3'-DIDEOXYNUCLEOSIDES ET DE 2',3'-DIDEOXYNUCLEOSIDES

(30) Priority: 05.08.2003 IT MI20031610
(43) Date of publication of application: 10.05.2006
(73) Proprietor: Archimica S.r.l., 20124 Milano (IT)
(72) Inventor: BERTOLINI, Giorgio, I-20099 Sesto San Giovanni (IT); DELEO, Maurizio, I-20152 Milano (IT); VELATI, Maurizio, I-27030 Mezzana Rabattone (IT); FRIGERIO, Marco, I-20147 Milano (IT); CASTOLDI, Patrizia, I-20020 Lainate (IT)
(74) Representative: Pistolesi, Roberto
(86) International application number: PCT/IB2004/002520
(87) International publication number: WO 2005/012325

(56) References cited:
- US-A- 5 290 927
- US-A- 5 466 793
- CHU, C. K. ET AL: "General syntheses of 2',3'-dideoxynucleosides and 2',3'-didehydro-2',3'-dideoxynucleosides" JOURNAL OF ORGANIC CHEMISTRY CODEN: JOCEAH; ISSN: 0022-3263, vol. 54, no. 9, 1989, pages 2217-2225, XP002304765 cited in the application

## Description

The present invention relates to a process for preparing 2',3'-didehydro-2',3'-dideoxynucleosides and 2',3'-dideoxynucleosides, and more particularly relates to a process for preparing these compounds that comprises the reductive elimination of 2',3'-dideoxy-2'(3')-(halo)-3'(2')-acylnucleosides with zinc and a suitable activating agent, the said process being characterized by a novel procedure for the removal of divalent zinc from the reaction medium, which is particularly advantageous from an industrial viewpoint.

### FIELD OF THE INVENTION

2',3'-Didehydro-2',3'-dideoxynucleosides and 2',3'-dideoxynucleosides form part of an important therapeutic category, the category of "nucleoside mimetics", which are generally endowed with antitumoral and antiviral activity. From a structural viewpoint, these compounds are characterized by a number of changes in the sugar portion of the nucleoside, in particular by the absence of hydroxyl groups in the 2',3' positions (dideoxy) and by the optional presence of a 2',3' double bond (didehydro).

Examples of the 2',3'-didehydro-2',3'-dideoxynucleoside category that may be mentioned include Stavudine (Merck Index, No. 8881, 2001), 5-fluoro-2',3'-dideoxy-2',3'-didehydro-β-D-cytidine (β-D-Fd4C) (Bioorganic & Medicinal Chemistry Letters (1998), 8, 3245-3250) and 5-fluoro-2',3'-dideoxy-2',3'-didehydro-β-L-cytidine (β-L-Fd4C) (Bioorganic & Medicinal Chemistry Letters (1998), 8, 3245-3250).

In addition to having advantageous intrinsic pharmaceutical properties, 2',3'-didehydro-2',3'-dideoxynucleosides are also used as intermediates in the preparation of other categories of nucleoside mimetics, for example 2',3'-dideoxynucleosides such as didanosine (Merck Index No. 3125, 2001), dideoxyadenosine (Merck Index No. 3128, 2001), and zalcitabine (Merck Index No. 10163, 2001), which may be obtained by reducing the 2', 3' double bond of the corresponding 2',3'-didehydro precursors, as reported, for example, in J. Org. Chem. (1989), 54, 2217-2225 and in J. Org. Chem. (1989) 54, 4780-4785.

The literature discloses various methods for synthesizing 2',3'-didehydro-2',3'-dideoxynucleosides.

Of particular importance in the present context are methods for the reductive elimination of suitably protected 2',3'-dideoxy-2'(3')-(halo)-3'(2')-acylnucleosides, by reaction with zinc and a suitable activating agent.

Examples of these processes are found in EP 334 368 (Mansuri et al.), in which is described the synthesis of 2',3'-didehydro-2',3'-dideoxynucleosides by reduction with zinc and copper, in US 5 466 793 (Ajinomoto) by reaction with zinc and acetic acid, and in Italian patent application MI2000A000810 (Archimica S.p.A.), in which is reported the preparation of stavudine and precursors thereof, via the reductive elimination mentioned above performed with zinc and ammonium or phosphonium salts.

In all these reactions, irrespective of the activating system used, divalent zinc is formed as a by-product of the redox reaction.

As is well known to those skilled in the art, it is necessary to minimize the presence of divalent zinc in the final 2',3'-didehydro-2',3'-dideoxynucleoside, both in the case where the said product represents the therapeutically active molecule, on account of possible toxicological implications, and in the case where it is used as an intermediate and subjected to catalytic hydrogenation, on account of the poisoning action of zinc on the hydrogenation catalyst, as discussed in US 5 466 793 and US 5 290 927.

Although the processes mentioned above make it possible to obtain the desired products in satisfactory yields, they are of limited industrial applicability precisely because of the procedures for removing the divalent zinc used. In particular, to obtain a final product of satisfactory purity in EP 334 368 A2 (D4U synthesis, page 16), use is made of chromatographic purification of the worked-up crude product, while in patent application MI2000A000810, a rather complex work-up is performed.

More generally, the methods reported in the literature for the removal of the divalent zinc from products of this type involve:
- chromatographic techniques (Acta Chem. Scand. B 36 (1982) 251-253)
- precipitation of the divalent zinc as zinc hydroxide by adding large amounts of a basic ion-exchange resin, for instance Amberlyst 27 (usually 20-30 ml of resin per gram of product), followed by subsequent filtration of the zinc hydroxide (J. Org. Chem. (1989) 54, 4780-4785). The filtration of the zinc hydroxide often proves to be difficult due to the formation of a gel;
- extraction of the divalent zinc by aqueous washing with chelating agents, in particular with EDTA. In this case, appreciable amounts of EDTA are required (about 10 grams of EDTA per gram of product) to remove all of the zinc (Nucleosides & Nucleotides (1996) 15, 47-58). In addition, this procedure cannot be used if the product is soluble or partially soluble in water on account of the substantial losses of product into the aqueous phase.

In conclusion, the methods listed above are particularly laborious and/or cannot be conveniently applied on a large scale and the removal of the divalent zinc represents at the present time one of the main unresolved problems in the industrial use of this synthetic route, which is otherwise advantageous, for preparing 2',3'-didehydro-2',3'-dideoxynucleosides.

We have now found a novel process for removing the divalent zinc in reactions of this type, which is particularly simple, efficient and easy to apply industrially, which makes the preparation of 2',3'-didehydro-2',3'-dideoxynucleosides very advantageous relative to the methods described in the literature.

### SUMMARY OF THE INVENTION

The subject of the present invention is thus a process for preparing 2',3'-didehydro-2',3'-dideoxynucleosides of formula 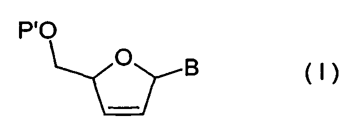 in which
- P': represents hydrogen or a suitable protecting group P, and
- B: represents a natural or modified, optionally substituted purine or pyrimidine base or a five- or six-membered monocyclic or eleven- or twelve-membered bicyclic, optionally substituted heterocyclic system containing at least one nitrogen atom;
which comprises the reductive elimination reaction of the compound of formula 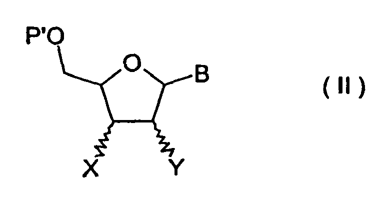 in which
- X and Y: represent, alternately, a halogen or an acyloxy group RCOO-,
- P' and B: have the meanings given above,
by reaction with zinc metal and a suitable activating agent,
characterized in that the divalent zinc is removed by precipitation, from an organic phase, of the corresponding zinc sulfide, by addition of a solution of an alkali metal or alkaline-earth metal sulfide to the said organic phase.

### DETAILED DESCRIPTION OF THE INVENTION

The process for preparing the 2',3'-didehydro-2',3'-dideoxynucleosides of formula I, which is the subject of the present invention, comprises the reductive elimination reaction of a compound of formula II.

In the compounds of formula I and of formula II above, the substituents P', P, B, X and Y have the following meanings:
P' represents a hydrogen or a protecting group P for the hydroxyl function, which is resistant to the reduction conditions used herein, as described, for example, by Theodora W. Greene, Protective Groups in Organic Synthesis; P' preferably represents a protecting group P.

Preferably, P represents a benzyl or trityl, which are optionally substituted, or a silyl group that is stable under mildly acidic conditions, for instance tert-butyldimethylsilyl or tert-butyldiphenylsilyl, or an acyl RCO-, in which R represents H, an alkyl R¹, an optionally substituted aryl Ar or a group R¹COOC(R²R³)-, in which R¹, R² and R³ represent H or a linear or branched C₁-C₁₁ alkyl.

Preferably, P represents an acyl group RCO- in which R represents a C₁-C₅ alkyl R¹, preferably a methyl or ethyl, or an optionally substituted aryl Ar, preferably a p-methylphenyl, or a group R¹COOC(R²R³)- in which R¹, R² and R³ represent a C₁-C₅ alkyl, preferably a methyl;

B represents a purine or pyrimidine base, in natural form or optionally substituted, for example fluorinated or methylated, or modified on the ring, for example aza-substituted, or a five-membered monocyclic heterocyclic system, for instance an imidazole, a pyrazole or a triazole, which are optionally substituted, or a six-membered monocyclic heterocyclic system, for instance an optionally substituted triazine, or an 11- or 12-membered bicyclic heterocyclic system (6,5 and 6,6 systems), for instance an optionally substituted benzimidazole, which contain at least one nitrogen atom. Preferred bases are natural or optionally substituted, for example fluorinated or methylated, purine or pyrimidine bases, for instance adenosine, inosine, 5-fluorocytidine and methyluridine;

X and Y represent, alternately, a halogen chosen from chlorine, bromine and iodine, and an acyloxy group RCOO-, in which R has the meanings given above.

Preferably, X and Y represent, alternately, bromine and an acyloxy group RCOO-, in which R represents an alkyl R¹ chosen from methyl and ethyl, preferably methyl, or a p-methylphenyl, or a group R¹COOC(R²R³)- in which R¹, R² and R³ represent methyl.

Examples of compounds of formula II that are particularly preferred are the compounds in which X and Y represent, alternately, a bromine and an acyloxy group RCOO-, in which R preferably represents a CH₃ group or a CH₃COOC(CH₃)₂- group, P' represents a protecting group for acyl RCO-, in which R represents a CH₃ group or a CH₃COOC(CH₃)₂- group and B represents adenine, 5-F-cytosine, inosine, hypoxanthine or thymine. According to the present invention, the compounds of formula II that are particularly preferred are those in which the group P and the group X (or Y) are identical.

The precursors of formula II may be prepared according to methods described in the literature, for example as reported in US 5 290 927 or in EP 334 368, which are incorporated herein by reference.

The reductive elimination reaction of the compounds of formula II to give the compounds of formula I of the present process is performed according to conditions already described in the art, by reaction with zinc metal and a suitable activating agent, for instance copper (EP 334 368) or acetic acid (J. Org. Chem. (1989) 54, 4780-4785) or, preferably, ammonium or phosphonium salts (MI2000A000810).

This last variant is particularly preferred since it allows the products of formula I to be obtained in higher yields and with less formation of by-products compared with the other known methods, and without using other potentially toxic metals that are difficult to dispose of. However, the method for removing divalent zinc, which is the subject of the present invention, maintains its general applicability in reductive elimination processes of this type irrespective of the activating agent used.

The general experimental conditions of the reductive elimination reaction mentioned above involve the use of solvents such as tetrahydrofuran, dimethylacetamide, alcohols (methanol, ethanol or isopropanol), acetonitrile, chlorinated solvents (methylene chloride), dimethyl sulfoxide or mixtures of these solvents (for example acetonitrile-methylene chloride), zinc (from 1 to 5 equivalents and preferably from 2 to 4 equivalents) and an activating agent such as copper metal or carboxylic acids, for instance acetic acid, or, preferably, ammonium or phosphonium salts (from 0.1 to 3 equivalents and preferably from 0.5 to 1.5 equivalents) at a temperature generally of between 0°C and 60°C and preferably between 20°C and 30°C. The general experimental conditions and, in particular, the equivalence of reagents and the reaction temperature that are preferred vary depending on the substrate and the solvent used, and are not intended to limit the scope of the present invention.

The process according to the present invention is characterized by the removal, by precipitation as zinc sulfide, of the divalent zinc from an organic phase, preferably from the reaction medium, by adding a solution, preferably an aqueous solution, of alkali metal and alkaline-earth metal sulfides, which are preferably water-soluble, for instance sodium sulfide, at a temperature of between 0°C and 60°C and preferably between 15°C and 30°C.

The organic phase, preferably the reaction medium, generally consists of solvents such as tetrahydrofuran, dimethylacetamide, alcohols, for example methanol, ethanol or isopropanol, acetonitrile, chlorinated solvents such as methylene chloride, dimethyl sulfoxide or mixtures of these solvents, for example acetonitrile-methylene chloride mixtures, preferably tetrahydrofuran or a mixture of acetonitrile and methylene chloride.

The sulfide solution comprises a polar solvent generally chosen from aprotic dipolar solvents, for instance dimethylformamide or dimethyl sulfoxide and water, preferably water, and the selected sulfide in an amount of at least one equivalent relative to the starting material, preferably in slight excess.

Preferred mineral sulfides are alkali metal or alkaline-earth metal sulfides that are highly water-soluble, such as sodium sulfide, potassium sulfide and lithium sulfide, and preferably sodium sulfide.

The sulfide solution is generally prepared by dissolving the selected sulfide in a minimum amount of solvent at a temperature generally of between 0°C and 100°C and preferably between 40°C and 60°C.

In the preferred embodiment, the divalent zinc is removed by precipitation of the zinc sulfide by adding an aqueous solution of sodium sulfide (dissolved at 45-55°C) directly to the reaction solution in tetrahydrofuran or in a mixture of acetonitrile and methylene chloride maintained at a temperature of between 15°C and 30°C.

The removal of the zinc sulfide thus formed may be performed according to standard techniques, for instance centrifugation, decantation or filtration.

Examples of preferred compounds of formula I (P' = P) that may be obtained according to the process of the present invention are 5'-acyl-2',3'-didehydro-2',3'-dideoxyadenosine, 5'-acyl-2',3'-didehydro-2',3'-dideoxyinosine, 5'-acyl-2',3'-didehydro-2',3'5-fluorocytidine, 5'-acyl-2',3'-didehydro-2',3'-cytidine, 5'-acyl-2',3'-didehydro-2',3'-dideoxymethyluridine, even more preferably 5'-(2-acetoxybutyryl)-2',3'-didehydro-2',3'-dideoxy-5-fluorocytidine (formula I, P = CH₃COOC(CH₃)₂CO-, B = 5-fluorocytosine), 5'-(2-acetoxybutyryl)-2',3'-didehydro-2',3'-dideoxyadenosine (formula I, P = CH₃COOC(CH₃)₂CO-, B = adenine), 5'-(2-acetoxybutyryl)-2',3'-didehydro-2',3'-dideoxyinosine (formula I, P = CH₃COOC(CH₃)₂CO-, B = hypoxanthine) and 5'-acetylstavudine (formula I, P = CH₃CO, B = thymine).

The compound of formula I obtained in accordance with the present invention may then be subjected to simple deprotection, thus giving the corresponding deprotected 2',3'-didehydro-2',3'-dideoxynucleoside (formula I, P' = H), according to methods that are well known in the art, or to subsequent reactions, without the need for further uneconomical and complex purification treatments that are of little industrial applicability. One particularly preferred aspect of the present invention involves the preparation of 5-fluoro-2',3'-dideoxy-2',3'-didehydro-β-D-cytidine (formula I, P' = H, B = 5-fluorocytidine), stavudine (P' = H, B = thymine) by simple deprotection, optionally as a one-pot reaction, of the corresponding 5'-protected 2',3'-dideoxy-2',3'-didehydronucleoside (formula I, P' = P), prepared according to the invention.

Alternatively, the compound of formula I, preferably in protected form (P = P'), may be subjected to subsequent reactions, preferably to reduction of the 2',3' double bond, under standard conditions, optionally as a one-pot reaction, giving in this case, after 5' deprotection, the corresponding 2',3'-dideoxynucleosides (formula II, X = Y = P' = H).

Preferred examples of these compounds (formula II, P' = H, X = Y = H), that may be obtained by reductive elimination, removal of the zinc according to the present invention, subsequent reduction of the 2',3' double bond and final deprotection as described above are dideoxyadenosine (ddA, II, P' = X = Y = H, B = adenine), didanosine (II, P' = X = Y = H, B = inosine) and zalcitabine (II, P' = X = Y = H, B = cytidine).

The experimental tests that follow are now given for the purpose of illustrating the present invention more clearly, without, however, wishing to limit it.

### EXAMPLES

### EXAMPLE 1

### 5-Fluoro-2',3'-dideoxy-2',3'-didehydro-β-D-cytidine (β-D-Fd4C)

2-Acetoxybutyryl bromide (56 ml) is added dropwise at 5°C to a suspension of 5-fluorocytidine (25 g) in ethyl acetate (195 ml) and acetonitrile (47 ml). The reaction mixture is stirred at 15-20°C overnight, the solution is then cooled to 5°C and a solution of potassium bicarbonate (63 g) in water (290 ml) is added, while keeping the temperature below 15°C. The reaction mixture is stirred at 25°C for 30 minutes, while checking that the aqueous phase has a pH of about 8. The phases are separated and the aqueous phase is extracted with ethyl acetate (100 ml). The combined organic phases are washed with a 15% solution of sodium chloride in water (125 ml) and dried over anhydrous magnesium sulfate. The solvent is evaporated off under reduced pressure at about 30°C and the residue is then taken up in anhydrous tetrahydrofuran (250 ml). Zinc powder (12.5 g) and tributylamine hydrobromide (12.8 g) are added at about 20°C. The temperature of the reaction mass rises slowly to 27-30°C, and the reaction mixture is then stirred at 35°C for 3 hours.

The solid present is gradually filtered off and washed with tetrahydrofuran (50 ml), and a solution of sodium sulfide nonahydrate (23 g) dissolved at 50°C in water (12 ml) is added. The suspension thus obtained is stirred for about 1 hour at 20-25°C, the zinc sulfide is then filtered off and the clear solution is evaporated under reduced pressure at 30-35°C.

The residue is taken up in methanol (80 ml) and the solvent is evaporated off under vacuum. The residue is redissolved in methanol (200 ml), 30% sodium methoxide in methanol (1.7 g) is added and the reaction mixture is stirred overnight at 20-25°C.

About 100 ml of methanol are evaporated off under reduced pressure and the solid obtained is then filtered off, washing with cold methanol (15 ml). The product obtained is dried to give about 10 g of crude dry 5-fluoro-2',3'-dideoxy-2',3'-didehydro-β-D-cytidine (β-D-Fd4C). The product is dissolved in methanol (400 ml) at 35°C and the insoluble material is then gradually filtered off. The solution thus obtained is evaporated under reduced pressure at 35°C to about 60 ml. The suspension is cooled to 0-5°C and stirred at this temperature for about 1 hour. The solid is filtered off, washed with cold methanol (10 ml) and dried under vacuum at 30°C to give 8.9 g of pure 5-fluoro-2',3'-dideoxy-2',3'-didehydro-β-D-cytidine (β-D-Fd4C) (41% yield). m.p.: 185-186°C.

### EXAMPLE 2

### Dideoxyadenosine (ddA)

Adenosine (10 g) is suspended in acetonitrile (100 ml) and 2-acetoxybutyryl bromide (20.5 ml) dissolved in acetonitrile (80 ml) is added dropwise slowly at 20-25°C. At the end of the addition, the reaction mixture is stirred for about 2 hours. A solution of potassium bicarbonate (45 g) in water (180 ml) and methylene chloride (150 ml) are then added dropwise to the reaction mixture. The phases are separated and the aqueous phase is extracted with methylene chloride (50 ml). The combined organic phases are concentrated under reduced pressure. The residue is dissolved in tetrahydrofuran (100 ml) and zinc powder (5 g) and tributylamine hydrobromide (5.1 g) are added at about 20°C. The temperature of the reaction mass rises slowly to 27-30°C, and the reaction mixture is then stirred at 35°C for 3 hours.

The solid present is gradually filtered off, washing with tetrahydrofuran (20 ml) and a solution of sodium sulfide monohydrate (9.2 g), dissolved at 50°C in water (4.8 ml) is added. The suspension thus obtained is stirred for about 1 hour at 20-25°C, the zinc sulfide is then filtered off and the clear solution is evaporated under reduced pressure at 30-35°C. The residue is taken up in isopropanol (50 ml) and the solvent is evaporated off under vacuum. The residue is suspended in isopropanol (150 ml), 5% palladium-on-charcoal (3 g) is added and the mixture is hydrogenated at 2-3 bar and 50°C for 2 hours. The catalyst is gradually filtered off, washing with isopropanol (75 ml), 30% sodium hydroxide (9 ml) is then added at 20-25°C and the reaction mixture is stirred at 20-25°C for 2 hours. The insoluble material thus formed is gradually filtered off and the clear solution in isopropanol is concentrated under reduced pressure. The residue is redissolved in refluxing ethanol (90 ml) and the product is then left to crystallize at room temperature, after which it is cooled to 0-5°C and the suspension is stirred at this temperature for 1 hour. The solid is filtered off, washed with cold ethanol (10 ml) and dried under vacuum to give 3 g of dideoxyadenosine (ddA) (38% yield) m.p.: 182-183°C

### EXAMPLE 3

### Didanosine (ddl)

Inosine (10 g) is suspended in acetonitrile (180 ml) and 2-acetoxybutyryl bromide (20.5 ml) dissolved in acetonitrile (40 ml) is added dropwise slowly at 20-25°C. At the end of the addition, the reaction mixture is stirred for about 3 hours. A solution of potassium bicarbonate (45 g) in water (180 ml) and methylene chloride (150 ml) are then added dropwise to the reaction mixture. The phases are separated and the aqueous phase is extracted with methylene chloride (50 ml). The combined organic phases are concentrated under reduced pressure. The residue is dissolved in tetrahydrofuran (160 ml) and zinc powder (5 g) and tributylamine hydrobromide (10 g) are added at about 20°C. The temperature of the reaction mass rises slowly to 27-30°C and the reaction mixture is then stirred at 35°C for 3 hours.

The zinc present is gradually filtered off, washing with tetrahydrofuran (20 ml) and a solution of sodium hydride nonahydrate (9.2 g) dissolved at 50°C in water (4.8 ml) is added. Anhydrous magnesium sulfate (10 g) is added and the suspension thus obtained is stirred for about 1 hour at 20-25°C, and the zinc sulfide is then filtered off.

Concentrated sodium hydroxide (10 ml) is added to the tetrahydrofuran solution and the reaction mixture is stirred at 20-25°C for 1 hour, to give a precipitate. The tetrahydrofuran is separated off from the solid and this solid is suspended in methanol (200 ml). 5% palladium-on-charcoal (5.5 g) is added and the mixture is hydrogenated at 2-3 bar. The catalyst is gradually filtered off and the solvent is evaporated off under reduced pressure. The residue is dissolved in water (50 ml) and the solution is brought to about pH 7 with dilute hydrochloric acid. The resulting solution is evaporated to dryness under reduced pressure and the residue is suspended in isopropanol (50 ml). The salts are gradually filtered off and the solution is concentrated to about 20 ml. The suspension thus obtained is cooled to 0-5°C and stirred at this temperature overnight. The solid is filtered off, washed with isopropanol (5 ml) and dried under vacuum to give 2 g of didanosine (23 % yield). m.p.: 177-178°C.

### EXAMPLE 4

### 5'-Acetylstavudine (5'-Acd4T)

Zinc powder (16 g) and a solution of 2'-bromo-3',5'-diacetylthymidine (50 g) prepared as described in patent application MI2000A000810 in tetrahydrofuran (710 ml) and dimethyl sulfoxide (40 ml) are added together. Tributylamine hydrobromide (49 g) is then added. The temperature of the reaction mass rises slowly to 27-30°C, and the reaction mixture is then stirred at 35°C for 3 hours.

The zinc present is gradually filtered off, washing with tetrahydrofuran (100 ml) and a solution of sodium sulfide nonahydrate (29.6 g) dissolved at 50°C in water (15.5 ml) is added. Anhydrous magnesium sulfate (125 g) is added and the suspension thus obtained is stirred for about 6 hours at 20-25°C, and the zinc sulfide is then filtered off. The solution thus obtained is evaporated under reduced pressure, the residue is taken up in isopropanol (230 ml) and the suspension is stirred at 40°C for 30 minutes and then at 20-25°C for 4 hours. The solid thus obtained is filtered off and washed with isopropanol (50 ml). The crude wet product is dissolved in hot isopropanol (580 ml) and cooled to 20-25°C, and the suspension is stirred at this temperature for 1 hour. The solid is filtered off, washed with isopropanol (50 ml) and dried under vacuum at 40°C to give 18 g of 5'-acetylstavudine (55% yield). m.p.:179-180°C.

## Claims

1. Process for preparing 2',3'-didehydro-2',3'-dideoxynucleosides of formula 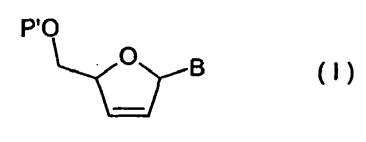 in which
P' represents hydrogen or a suitable protecting group P, and
B represents a natural or modified, optionally substituted purine or pyrimidine base or a five- or six-membered monocyclic or eleven- or twelve-membered bicyclic, optionally substituted heterocyclic system containing at least one nitrogen atom;
which comprises the reductive elimination reaction of the compound of formula 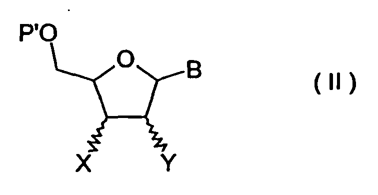 in which
X and Y represent, alternately, a halogen or an acyloxy group RCOO-,
P' and B have the meanings given above,
by reaction with zinc metal and a suitable activating agent,
**characterized in that** the divalent zinc is removed by precipitation, from an organic phase, of the corresponding zinc sulfide, by addition of a solution of an alkali metal or alkaline-earth metal sulfide to the said organic phase.

2. Process according to Claim 1, in which:
P' represents an acyl group RCO-, in which R represents a C₁-C₅ alkyl R¹, preferably a methyl, or a group R¹COOC(R²R³)-, in which R¹, R² and R³ represent a C₁-C₅ alkyl, preferably a methyl;
B represents an optionally substituted natural purine or pyrimidine base, preferably adenine, inosine, 5-F-cytosine, hypoxanthine or thymine;
X and Y represent, alternatively, bromine and an acyloxy group RCOO-, in which R represents a C₁-C₅ alkyl R¹, preferably methyl, or a group R¹COOC(R²R³)-, in which R¹, R² and R³ represent a C₁-C₅ alkyl R¹, preferably a methyl.

3. Process according to Claim 1, in which the said activating agent is chosen from copper, acetic acid and ammonium or phosphonium salts, preferably ammonium or phosphonium salts.

4. Process according to Claim 1, in which the said organic phase is chosen from solvents such as tetrahydrofuran, dimethylacetamide, alcohols, acetonitrile, chlorinated solvents and dimethyl sulfoxide, and mixtures thereof.

5. Process according to Claim 1, in which the said sulfide solution comprises a polar solvent chosen from dipolar aprotic solvents and water, preferably water.

6. Process according to Claim 1, in which the said sulfide solution comprises the chosen alkali metal or alkaline-earth metal sulfide in an amount of at least one equivalent relative to the starting material, preferably in slight excess.

7. Process according to Claim 1, in which the said mineral sulfide is an alkali metal or alkaline-earth metal sulfide, preferably sodium sulfide.

8. Process according to Claim 1, in which the precipitated zinc sulfide is removed by filtration.

9. Process according to Claim 1, which further comprises the reduction reaction of the double bond of the compound of formula I to give the corresponding 2',3'-dideoxynucleoside of formula 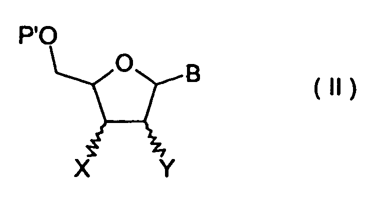 in which X = Y = H, and P' and B have the meanings given above.

10. Process according to Claim 1, which further comprises the deprotection reaction of a compound of formula 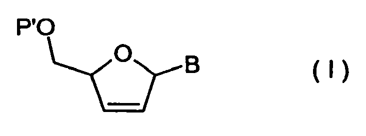 in which P' represents a protecting group P, and B has the meanings given above,
to give the corresponding compound of formula I,
in which P' represents hydrogen.

11. Process according to Claim 9, which further comprises the deprotection reaction of a compound of formula 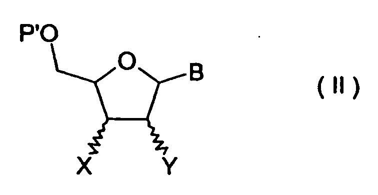 in which P' represents a protecting group P, X and Y represent H, and B has the meanings given above,
to give the corresponding compound of formula II,
in which P' represents hydrogen.

12. Process for preparing 5-fluoro-2',3'-dideoxy-2',3'-didehydro-β -D-cytidine, stavudine, dideoxyadenosine, didanosine and zalcitabine, which comprises a process according to Claims 1 to 10.

## Patentansprüche

1. Verfahren zur Herstellung von 2',3'-Didehydro-2',3'-dideoxynucleosiden der Formel I 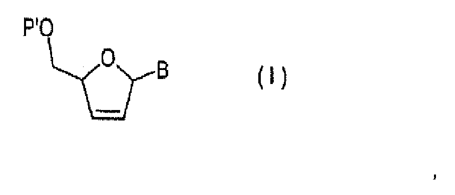 worin
P' für Wasserstoff oder eine geeignete Schutzgruppe P steht und
B für eine natürliche oder modifizierte, optional substituierte Purinbase oder Pyrimidinbase oder einen fünfgliedrigen oder sechsgliedrigen Monocyclus oder einen elfgliedrigen oder zwölfgliedrigen Bicyclus besteht, wobei das optional substituierte heterocyclische System wenigstens ein Stickstoffatom enthält,
durch reduktive Eliminationsreaktion der Verbindung der Formel II 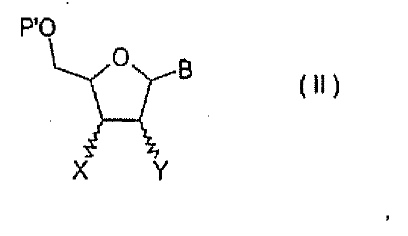 worin
X und Y alternierend für Halogen oder eine Acyloxygruppe RCOO- stehen, und
P' und B die oben angegebenen Bedeutungen haben, durch Umsetzung mit Zinkmetall und einem geeigneten Aktivator,
**dadurch gekennzeichnet, dass** das divalente Zink entfernt wird durch Präzipitation aus einer organischen Phase des entsprechenden Zinksulfids durch Zusatz einer Lösung eines Alkalimetallsulfids oder eines Erdalkalimetallsulfids zu der organischen Phase.

2. Verfahren nach Anspruch 1, worin
P' für eine Acylgruppe RCO- steht, worin R für C₁-C₅-Alkyl R¹, vorzugsweise Methyl, oder eine Gruppe R¹COOC(R²R³)- steht, worin R¹, R² und R³ für C₁-C₅-Alkyl, vorzugsweise Methyl, steht
B für eine optional substituierte natürliche Purinbase oder Pyrimidinbase, vorzugsweise Adenin, Inosin, 5-F-Cytosin, Hypoxanthin oder Thymin, steht, und
X und Y alternativ für Brom und eine Acyloxygruppe RCOO- stehen, worin R für C₁-C₅-Alkyl R¹, vorzugsweise Methyl, oder eine Gruppe R¹COOC(R²R³)- steht, worin R¹, R² und R³ für C₇-C₅-Alkyl R¹, vorzugsweise für ein Methyl, steht.

3. Verfahren nach Anspruch 1, worin der Aktivator ausgewählt ist aus Kupfer, Essigsäure, Ammoniumsalzen oder Phosphoniumsalzen, vorzugsweise aus Ammoniumsalzen oder Phosphoniumsalzen.

4. Verfahren nach Anspruch 1, worin die organische Phase ausgewählt ist aus Lösemitteln, wie Tetrahydrofuran, Dimethylacetamid, Alkoholen, Acetonitril, chlorierten Lösemitteln und Dimethylsulfoxid und Gemischen hiervon.

5. Verfahren nach Anspruch 1, worin die Sulfidlösung ein polares Lösemittel umfasst, das ausgewählt ist aus dipolaren aprotischen Lösemitteln und Wasser, vorzugsweise Wasser.

6. Verfahren nach Anspruch 1, worin die Sulfidlösung das ausgewählte Alkalimetallsulfid oder Erdalkalimetallsulfid in einer Menge umfasst, die wenigstens ein Äquivalent relativ zum Ausgangsmaterial umfasst, vorzugsweise einen geringen Überschuss.

7. Verfahren nach Anspruch 1, worin das Mineralsulfid ein Alkalimetallsulfid oder ein Erdalkalimetallsulfid ist, vorzugsweise Natriumsulfid.

8. Verfahren nach Anspruch 1, worin das präzipitierte Zinksulfid durch Filtration entfernt wird.

9. Verfahren nach Anspruch 1, weiter umfassend die Reduktionsreaktion der Doppelbindung der Verbindung der Formel I unter Erhalt des entsprechenden 2',3'-Dideoxynucleosids der Formel II 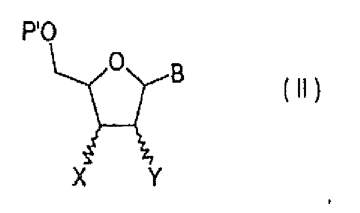 worin X und Y jeweils für H stehen und P' und B die oben angegebenen Bedeutungen haben.

10. Verfahren nach Anspruch 1, weiter umfassend die Schutzgruppenabspaltungsreaktion einer Verbindung der Formel I 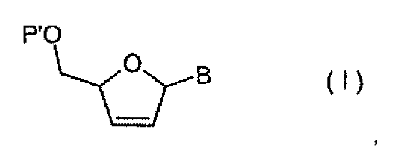 worin P' für eine Schutzgruppe P steht und B die oben angegebenen Bedeutungen hat,
unter Erhalt der entsprechenden Verbindung der Formel I, worin P' für Wasserstoff steht.

11. Verfahren nach Anspruch 9, weiter umfassend die Schutzgruppenabspaltungsreaktion einer Verbindung der Formel II 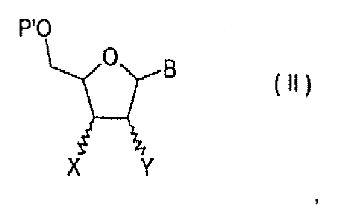 worin P' für eine Schutzgruppe P steht, X und Y jeweils für H stehen, und B die oben angegebenen Bedeutungen hat, unter Erhalt der entsprechenden Verbindung der Formel II, worin P' für Wasserstoff steht.

12. Verfahren zur Herstellung von 5-Fluor-2',3'-dideoxy-2',3'-didehydro-β-D-cytidin, Stavudin, Dideoxyadenosin, Didanosin und Zalcitabin, das ein Verfahren nach Anspruch 1 bis 10 umfasst.

## Revendications

1. Procédé de préparation de 2',3'-didéshydro-2',3'-didésoxynucléosides de formule 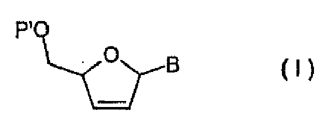 dans laquelle
P' représente un atome d'hydrogène ou un groupe protecteur P approprié, et
B représente une base purine ou pyrimidine éventuellement substituée, naturelle ou modifiée, ou un système hétérocyclique monocyclique à cinq ou six chaînons ou bicyclique à onze ou douze chaînons, éventuellement substitué, contenant au moins un atome d'azote ;
qui comprend la réaction d'élimination réductrice du composé de formule 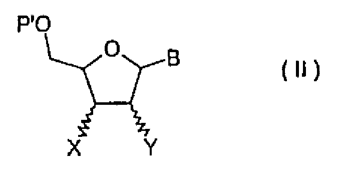 dans laquelle
X et Y représentent, alternativement, un atome d'halogène ou un groupe acyloxy RCOO-,
P' et B ont les significations données ci-dessus,
par réaction avec du zinc métallique et un agent d'activation convenable,
**caractérisé en ce que** le zinc divalent est séparé par précipitation, dans une phase organique, du sulfure de zinc correspondant, par addition d'une solution d'un sulfure de métal alcalin ou de métal alcalino-terreux à ladite phase organique.

2. Procédé selon la revendication 1, dans lequel :
P' représente un groupe acyle RCO-, dans lequel R représente un groupe alkyle en C₁-C₅ R¹, de préférence un groupe méthyle, ou un groupe R¹COOC(R²R³)-, dans lequel R¹, R² et R³ représentent un groupe alkyle en C₁-C₅, de préférence un groupe méthyle ;
B représente une base purine ou pyrimidine naturelle, éventuellement substituée, de préférence adénine, inosine, 5-F-cytosine, hypoxanthine ou thymine;
X et Y représentent, alternativement, un atome de brome et un groupe acyloxy RCOO-, dans lequel R représente un groupe alkyle en C₁-C₅ R¹, de préférence méthyle, ou un groupe R¹COOC(R²R³), dans lequel R¹, R² et R³ représentent un groupe alkyle en C₁-C₅ R¹, de préférence un groupe méthyle.

3. Procédé selon la revendication 1, dans lequel ledit agent d'activation est choisi parmi le cuivre, l'acide acétique et les sels d'ammonium ou de phosphonium, de préférence les sels d'ammonium ou de phosphonium.

4. Procédé selon la revendication 1, dans lequel ladite phase organique est choisie parmi des solvants tels que le tétrahydrofurane, le diméthylacétamide, les alcools, l'acétonitrile, les solvants chlorés et le diméthylsulfoxyde, et leurs mélanges.

5. Procédé selon la revendication 1, dans lequel ladite solution de sulfure comprend un solvant polaire choisi parmi les solvants aprotiques dipolaires et l'eau, de préférence l'eau.

6. Procédé selon la revendication 1, dans lequel ladite solution de sulfure comprend le sulfure de métal alcalin ou de métal alcalino-terreux choisi en quantité d'au moins un équivalent par rapport au composé de départ, de préférence en léger excès.

7. Procédé selon la revendication 1, dans lequel ledit sulfure minéral est un sulfure de métal alcalin ou de métal alcalino-terreux, de préférence le sulfure de sodium.

8. Procédé selon la revendication 1, dans lequel le sulfure de zinc précipité est éliminé par filtration.

9. Procédé selon la revendication 1, qui comprend en outre la réaction de réduction de la double liaison du composé de formule 1 pour donner le 2',3'-didésoxynucléoside correspondant de formule 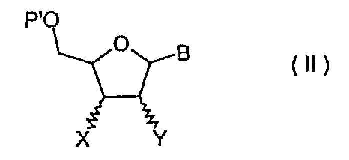 dans laquelle X = Y = H, et P' et B ont les significations données ci-dessus.

10. Procédé selon la revendication 1, qui comprend en outre la réaction de déprotection d'un composé de formule 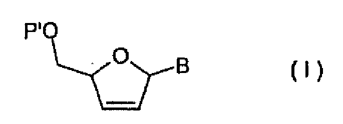 dans laquelle P' représente un groupe protecteur P, et B a les significations données ci-dessus,
pour donner le composé correspondant de formule I,
dans laquelle P' représente un atome d'hydrogène.

11. Procédé selon la revendication 9, qui comprend en outre la réaction de déprotection d'un composé de formule 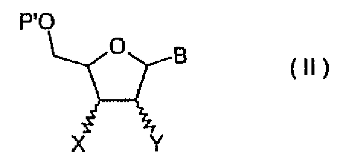 dans laquelle P' représente un groupe protecteur P, X et Y représentent H, et B a les significations données ci-dessus,
pour donner le compose correspondant de formule II,
dans laquelle P' représente un atome d'hydrogène.

12. Procédé de préparation de la 5-fluoro-2',3'-didésoxy-2',3'-didéshydro-β-D-cytidine, de la stavudine, de la didésoxyadénosine, de la didanosine et de la zalcitabine, qui comprend a procédé selon les revendications 1 à 10.
